**Europäisches Patentamt**

**European Patent Office**

**Office européen des brevets**

(19)

(11) Veröffentlichungsnummer: **0 031 135**
**B1**

(12) # EUROPÄISCHE PATENTSCHRIFT

(45) Veröffentlichungstag der Patentschrift:
18.03.87

(21) Anmeldenummer: **80107988.0**

(22) Anmeldetag: **17.12.80**

(51) Int. Cl.⁴: **A 61 B 5/12**

(54) **Elektroakustisches Messgerät.**

(30) Priorität: **21.12.79 DE 2951856**

(43) Veröffentlichungstag der Anmeldung:
**01.07.81 Patentblatt 81/26**

(45) Bekanntmachung des Hinweises auf die Patenterteilung:
**18.03.87 Patentblatt 87/12**

(84) Benannte Vertragsstaaten:
**AT GB IT**

(56) Entgegenhaltungen:
**DE-A-2 116 971**
**DE-A-2 855 794**
**FR-A-1 444 810**
**FR-A-2 438 463**
**US-A-3 404 235**

(73) Patentinhaber: **Siemens Aktiengesellschaft Berlin und München, Wittelsbacherplatz 2, D-8000 München 2 (DE)**

(72) Erfinder: **Zwicker, Eberhard, Prof. Dr., Walchstadter Strasse 38, D-8021 Icking (DE)**

## Beschreibung

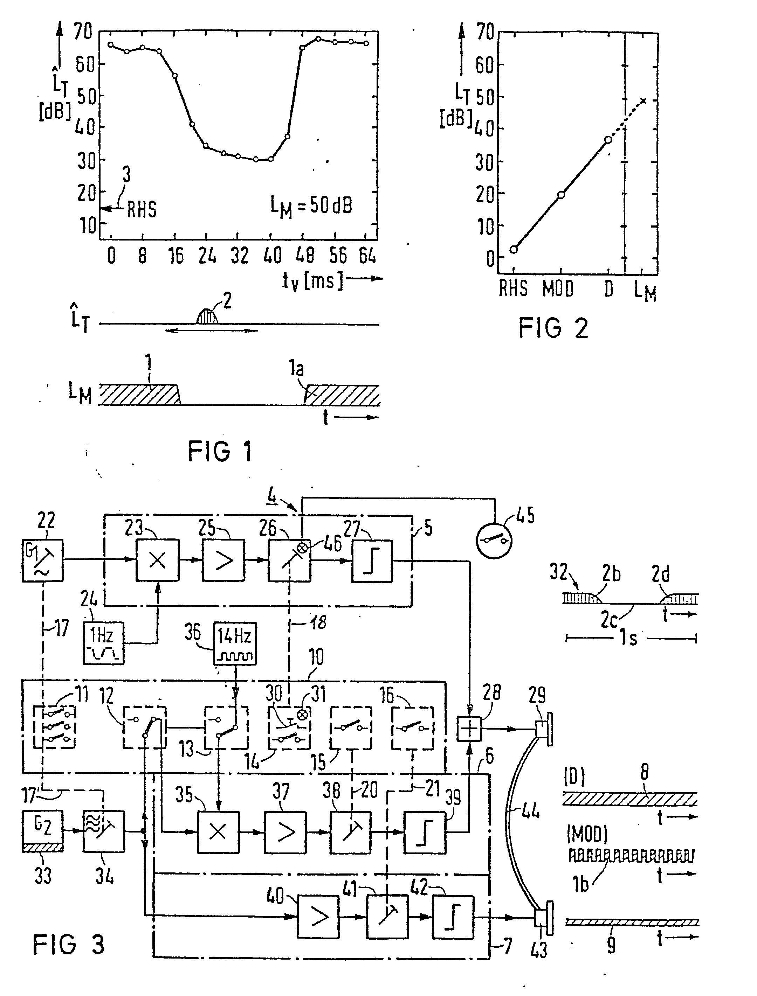

Die Erfindung betrifft ein elektroakustisches Meßgerät zur Prüfung des zeitlichen Auflösungsvermögens des Gehörs mittels dem zu untersuchenden Ohr über einen Hörer zuzuführenden Schallsignalen, die aus einem Testdauerton, der unterbrochen sein kann, und maskierendem Schall, der impulsmoduliert sein kann, bestehen, deren Tonhöhe und Pegel getrennt einstellbar sind. Ein Gerät dieser Art ist z.B. bekannt aus "ACUSTICA" Vol. 36 (1976/77), Seiten 173 bis 120, insbesondere Fig. 1.

Neben dem mit Audiometern in einfacher Weise bestimmbaren Frequenzauflösungsvermögen des Gehörs spielt auch sein Zeitauflösungsvermögen bei der Aufnahme von Information, insbesondere bei derjenigen von Spreche, eine sehr wichtige Rolle. Wenn das Gehör nach lauten Vokalen nicht rasch genug wieder so empfindlich wird, daß es auch nachfolgende leise Konsonanten noch wahrnehmen kenn, wird die Sprache nicht verstanden. Dieser Effekt ist auch von Normalhörenden bemerkbar, wenn ein Redner in einem stark nachhallenden Raum schnell spricht (z.B. die gelegentlich ganz unverständlichen ansagen auf Bahnhöfen). Die für die in der Sprache enthaltene Information sehr wichtigen, aber leisen Konsonanten werden dann von den nachhallenden lauten Vokalen verdeckt.

Das Zeitauflösungsvermögen des Gehörs kann durch Vor-Simultan- und Nachverdeckung beschrieben werden. Die Kombination aus den Wirkungen dieser Verdeckungen wird in den sog. Mithörschwellen-Periodenmustern von periodisch amplitudenmodulierten Maskierern ausgemessen, wie es in der in obigem Einleitungsabsatz angegebenen Literaturstelle behandelt ist. Dabei wird periodisch mit einer Modulationsfrequenz von z.B. 15,6 Hz rechteckförmig moduliert. Der Periode dieses maskierenden Schalls beträgt dann 64 ms (32 ms ein- und 32 ms ausgeschaltet). Innerhalb dieser Periode wird zu einem definierten (von Meßpunkt zu Meßpunkt veränderbaren) Zeitpunkt jeweils ein kurzer, z.B. 4 ms langer, Testtonimpuls dergeboten. Da sich die Perioden mit 15,6 Hz wiederholen, ist der Testschall eine Pulsfolge von Tonimpulsen der Folgefrequenz von 15,6 Hz. Verdeckender Schall und Testschall sind zeitlich synchronisiert: Zur Bestimmung des Mithörschwellen-Periodenmusters wird die Mithörschwelle des Testschalls als Funktion der zeitlichen Verschiebung des Testschalls innerhalb einer Periode des verdeckenden Schalls gemessen.

In der Fig. 1 ist das Ergebnis einer solchen Messung unter Verwendung eines Gerätes nach vorliegender Erfindung für ein verdeckendes Oktav-Rauschen der Mittenfrequenz von 1,5 kHz (rechteckförmig mit 15,6 Hz in der Amplitude vollständig moduliert) dargestellt.

Die Testtonfrequenz beträgt 1,5 kHz, die Testtondauer 4 ms bei 2 ms Anstiegs- und Abfallzeit. Der Pegel $L_T$, aus dem der gerade wahrnehmbare Testtonpuls $L_T$ ausgeschnitten wurde, ist in Abhängigkeit von der Verzögerungszeit $t_v$ innerhalb einer Periode des verdeckenden Schalls dargestellt. Um den untersuchten Zusammenhang ausreichend genau erfassen zu können, wurden 16 Meßpunkte innerhalb der Periode von 32 ms gewählt. In der Fig. 1 sind unterhalb der Abszisse eine Periode des verdeckenden Schalls 1, 1a (schräg schraffiert) sowie ein Testimpuls 2 (senkrecht schraffiert) dargestellt. Der Pegel $L_M$ des Oktav-Rauschens, aus dem der verdeckende Schall 1, 1a ausgeschnitten wurde, beträgt 50 dB. Das Mithörschwellen-Periodenmuster zeigt hohe Mithörschwellen in denjenigen Teilen der Periode, in denen der verdeckende Schall eingeschaltet ist. Die Mithörschwelle sinkt nach dem abschalten des verdeckenden Schalls 1 langsamer ab (Nachverdeckung) als sie vor dem Einschalten des verdeckenden Schalls 1a wieder ansteigt (Vorverdeckung). Die Ruhehörschwelle (RHS), die durch einen Pfeil 3 an der Ordinate gekennzeichnet ist, wird während der zwischen 1 und 1a liegenden Pause nicht erreicht. Vielmehr liegt das Minimum des Mithörschwellen-Periodenmusters bei den gewählten Parametern etwas unterhalb der Mitte zwischen dem Maximum und der Ruhehörschwelle (RHS).

Die Erfindung geht von der Erkenntnis aus, daß für die Charakterisierung des Zeitauflösungsvermögens weniger der Verlauf des Mithörschwellen-Periodenmusters im Detail entscheidend ist als vielmehr die Differenz zwischen dem Maximum und dem Minimum im Vergleich mit dem Wert der Ruhehörschwelle (RHS). Sie hat sich daher die Aufgabe gestellt, das zur Aufnahme eines vollständigen Musters in der psychoakustischen Meßtechnik bekannte aufwendige Gerät zu vermeiden und ein unkompliziertes sowie einfach zu bedienendes Meßgerät anzugeben, mit dem das Maximum und das Minimum bzw. ihre Differenz bestimmt werden können. Diese Aufgabe wird bei einem elektroakustischen Meßgerät nach dem Oberbegriff des Anspruchs 1 durch die im kennzeichnenden Teil dieses Anspruchs angegebenen Maßnahmen gelöst. Zweckmässige ausgestaltungen der Erfindung sind Gegenstand der Unteransprüche.

Eine Möglichkeit der Lösung ist, anstelle der 16 Punkte des gesamten Mithörschwellen-Periodenmusters lediglich die beiden für die Extrema maßgeblichen Werte bei $t_v = 0$ bzw. bei $t_v = 32$ ms zu bestimmen. Es darf jedoch nicht angenommen werden, daß das Minimum immer genau in der Mitte der Pause liegt, wie aus dem oben genannten Literaturzitat (vorletzte Zeile, Absatz 1 der Beschreibungseinleitung) entnommen werden kann. Das Minimum verschiebt sich häufig nach größeren Verzögerungszeiten hin.

Es wäre daher noch günstiger, wenn das Minimum mit einem Dauertestton ausgemessen werden könnte. Dies wäre erlaubt, falls eine Verschiebung der Mithörschwelle nach kleineren Pegeln als Folge der Integrationseigenschaften des Gehörs berücksichtigt werden könnte. Wird das Maximum ebenfalls mit einem Dauertestton bestimmt, so ist das leicht möglich, weil dann nur die Differenz zwischen Maximum und Minimum wichtig ist. Das Maximum läßt sich, wie Versuche, die zur Erfindung führten, zeigten, mit einem Dauertestton bestimmen, wenn die Pause im amplitudenmodulierten verdeckenden Oktav-Rausch abgeschaltet und der verdeckende Schall ebenso wie der Testschall als Dauerschall dargeboten werden.

Eine solche Methode hätte zusätzlich den großen Vorteil, daß der Patient immer denselben Testton zu beachten hätte. Dieser wird, ohne daß er für die Messung seine Wirkung als Dauerton verliert, wegen der dann leichteren Identifizierbarkeit hinsichtlich Frequenz und Pegel zweckmäßigerweise unterbrochen dargeboten (z.B. 500 ms ein, 500 ms aus). Von diesem im Vergleich zum impulsmodulierten Maskierton sehr langsam modulierten, d.h. unterbrochenen, Testton werden (a) die Ruhehörschwelle (RHS), (b) die Mithörschwelle (D) bei verdeckendem Oktav-Dauerrauschen, dessen Pegel so gewählt ist, daß die Mithörschwelle etwa 30 dB über der RHS liegt, und (c) die Mithörschwelle (MOD) bei verdeckendem amplitudenmoduliertem Oktav-Rauschen gemessen. Damit ergeben sich aus drei Testdauerton-Schallmessungen, die auch von ungeübten Personen leicht durchgeführt werden können, neben der Ruhehörschwelle (RHS) zwei weitere Meßwerte (D und MOD), die eine enge Beziehung zum Maximum bzw. zum Minimum des Mithörschwellen-Periodenmusters aufweisen und daher für das Zeitauflösungsvermögen des Gehörs charakteristisch sind.

Weitere Einzelheiten und Vorteile der Erfindung werden nachfolgend anhand der in den Figuren dargestellten Ausführungsbeispiele erläutert.

In der Fig. 1 ist das Mithörschwellen-Periodenmuster von 4 ms langen 1,5 kHz Tonimpulsen verdeckt durch Oktav-Rauschen bei 1,5 kHz Mittenfrequenz und einem Pegel von 50 dB dargestellt, das mit 15,6 Hz rechteckförmig moduliert ist,

in der Fig. 2 in einem Diagramm die unter den Bedingungen der Fig. 1 bestimmten Mithörschwellen von Dauertönen verdeckt durch Dauerrauschen (D) und durch moduliertes Rauschen (MOD) sowie die Ruhehörschwelle (RHS),

in der Fig. 3 das Blockschaltbild eines Gerätes und die damit erzielbaren Zeitfunktionen von Schallpegeln,

in der Fig. 4 in einem Diagramm die Frequenzabhängigkeit der Verstärkung g der zur Herstellung der bandbegrenzten Rauschen benutzten Filter,

in der Fig. 5 in Diagrammen zu "Zeitauflösungsgeraden" verbundene Mithörschwellen (D, MOD, RHS) bei Frequenzen von 500 Hz, 1500 Hz und 4000 Hz und verschiedenen Pegeln des maskierenden Rauschens,

in der Fig. 6 eine mit Fig. 5 übereinstimmende Auftragung der Mittelwerte von Messungen an 16 Versuchspersonen bei 500 Hz, 7500 Hz und 4000 Hz und einem maskierenden Rauschen (L) von 40 dB über RHS und

in der Fig. 7 die Auftragung von Messungen für eine Versuchsperson mit einem Hörverlust von etwa 50 dB bei Frequenzen um 4000 Hz unter sonst mit Fig. 6 übereinstimmenden Bedingungen.

Sollen die drei Meßwerte RHS, D, MOD für Normalhörende, wie in Fig. 2 angedeutet, auf einer Geraden liegen, wenn sie in gleichen horizontalen Abständen voneinander aufgetragen sind, und soll außerdem die Messung bei drei Frequenzwerten durchgeführt werden, so ist unter dem Gesichtspunkt einer möglichst lückenlosen Abdeckung aller möglichen Hörstörungen und einfacher graphischer Vorstellung folgende Festlegung der Variablen zweckmäßig:

Testschall

Unterbrochene Dauertöne (500 ms ein, 500 ms aus = 1 Hz) bei Frequenzen von 500 Hz, 1500 Hz und 4000 Hz, einstellbar in 5 dB-Schritten für Schalldruckpegel zwischen -5 dB und +115 dB. Zusätzlich kann zur genaueren Bestimmung der Schwelle noch eine Absenkung bzw. Anhebung der in den 5 dB-Schritten erreichbaren Werte um 2,5 dB vorgesehen sein.

Verdeckender (maskierender) Schall

Für den verdeckenden Schall ist zunächst für oktavbreites Dauerrauschen zur Bestimmung der Mithörschwelle D bei verdeckendem Dauerrauschen ein Generator vorzusehen für die Erzeugung von Rauschbändern mit den Mittenfrequenzen von 500 Hz, 1500 Hz und 4000 Hz, deren Pegel in 10 dB-Schritten für Schalldruckpegel zwischen 0 dB und 110 dB als Dauerschall einstellbar sind. Zur Bestimmung der MOD ist dasselbe Rauschen vorzusehen, jedoch zusätzlich ein Modulator zur rechteckförmigen Amplitudenmodulierung mit 14 Hz, so daß Perioden von 72 ms und Pausen von 36 ms entstehen.

In der Fig. 3 sind längs des Blockschaltbildes des Gerätes 4 sowie rechts zu den drei Kanälen, d.h. dem Testton-Kanal 5, dem Maskierungsschall-Kanal 6 und dem Vertäubungsschall-Kanal 7, gehörende Zeitfunktionen 2b, 2c, 8, 1b und 9 dargestellt. Das Gerät 4 enthält ein Bedienungsfeld 10 mit Tasten 11 bis 16 und dazugehörenden, in der Audiometrie an sich bekannten und der Übersichtlichkeit halber nicht gesondert dargestellten anzeigen der eingestellten Werte. Soweit zum Verständnis nötig, sind die Bedienungen zwischen den Elementen des Bedienungsfeldes 10 und den Kanälen 5 bis 7 mit gestrichelten Linien 17, 18, 20 und 21 in die Fig. 3 eingetragen.

Der Testschall wird im Kanal 5 von einem Tongenerator 22 aus erzeugt. Drei Frequenzen (500 Hz, 1500 Hz und 4000 Hz) sind einstellbar. Die Klirrfaktoren sind zur Vermeidung der Gefahr einer Verfälschung der Messung durch das Wahrnehmen von Verzerrungsprodukten kleiner als 1 ‰. Der nachfolgende Modulator 23, der von einem Generator 24 gesteuert wird, sorgt dafür, daß der Testton "weich" (etwa 20 ms Übergangszeit) mit einer Frequenz von etwa 1 Hz unterbrochen wird. Der unterbrochene Ton wird in einem Verstärker 25 verstärkt und über einem Teiler 26, einem Audiometer-Entzerrer 27 (vgl. DE-PS 28 55 794) und einem Summationspunkt 28 einer der beiden Kopfhörermuscheln 29 zugeführt. Der Teiler 26 wird vom Tastenfeld 10 aus gesteuert, so daß, wie beim jeweiligen Druck auf die Taste 14, der Pegel um 5 dB ansteigt bzw. abfällt. Der Schalldruckpegel $L_T$ wird in der bei Audiometern an sich bekannten Weise mittels Leuchtdioden angezeigt und ist zwischen -5 dB und +115 dB einstellbar. Der Testtonpegel $L_T$ kann durch den Druck auf eine zusätzliche Taste 30 um 2,5 dB abgesenkt werden, wobei eine Anzeigelampe 31 blinkt. Die Zeitfunktion des für alle drei Messungen gleichartigen Testschalls ist rechts neben dem Kanal 5 dargestellt und mit 32 bezeichnet. Er weist in dem dargestellten Stück von 1 Sekunde Länge einen auslaufenden Teil 2b, eine Pause 2c und einen beginnenden Teil 2d auf.

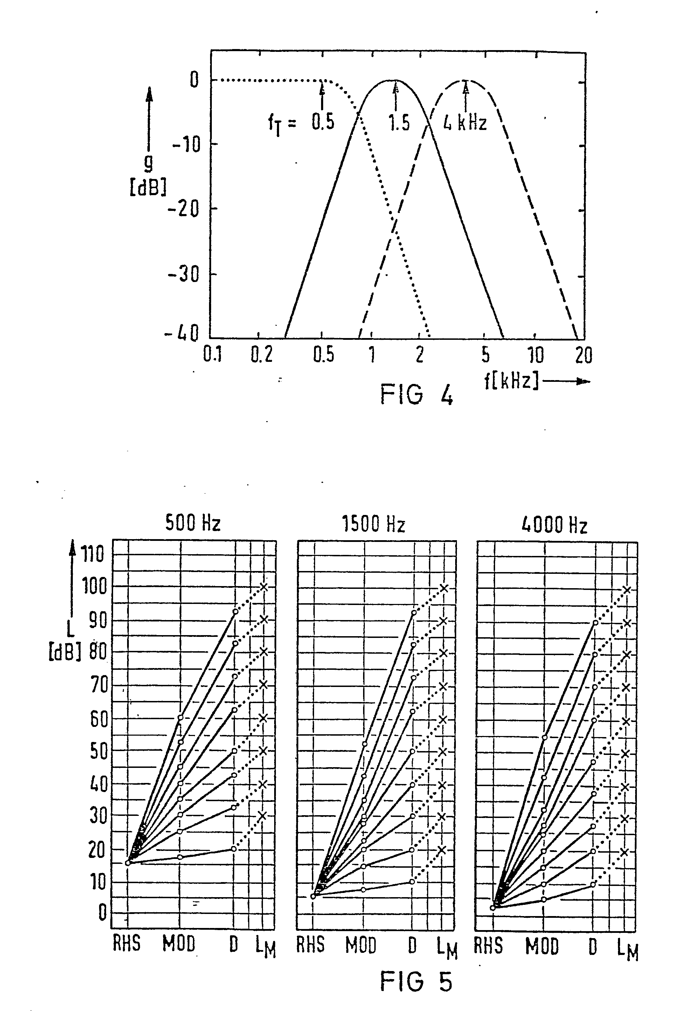

Der maskierende Schall des Kanals 6 wird in einem Generator 33 für weißes Rauschen erzeugt. Dem Generator 33 ist ein Tiefpaß 34 oder ein der an die gleiche Stelle zu bringenden Oktav-Bandpässe, die nicht gesondert dargestellt sind, nachgeschaltet. Die Umschaltung der Filter, deren Verstärkung g in Abhängigkeit von der Frequenz F in Fig. 4 dargestellt wird, ist mit der Umschaltung der Testtonfrequenzen in 11 über 17' gekoppelt.

Die Bandbreiten und die Dämpfungsflanken der Filter sind so gewählt, daß einerseits etwa infolge des Phasenganges der Filter auftretende störende Laufzeiten der Betriebsart MOD vermieden werden, andererseits aber der Lautstärkepegel $L_M$ des maskierenden Rauschens gegenüber demjenigen des weißen Rauschens des Generators 33 erheblich abgesenkt wird, damit das Gehör nicht stärker als für die Messung unbedingt erforderlich belastet wird. Dies ist insbesondere bei stark frequenzabhängigem Hörverlust für Patienten sehr angenehm, weil sonst im Falle einer breitbandigen Maskierung eine besonders große Gesamtlautstärke erzeugt wird. Für die tiefe Frequenzlage ($f_T = 500$ Hz) kann der Oktav-Bandpaß durch einen Tiefpaß 34 ersetzt werden.

Zur Messung der Ruhehörschwelle RHS bleibt im Kanal 6 das Rauschen durch Benutzung der Taste 12 abgeschaltet. Lediglich zur Messung der Mithörschwellen D bzw. MOD wird der Ausgang des Filters 34 an den Modulator 35 weitergeleitet. Für den Betriebszustand Modulation MOD wird der Modulator 35 mit dem 14 Hz-Rechteckgenerator 36 verbunden, so daß das maskierende Rauschen vollständig in der Amplitude moduliert wird.
Rechteckamplitudenmodulation wird benutzt, weil die Ergebnisse leichter interpretierbar sind. Die Modulation mit 14 Hz hat sich bewährt. Dann ergeben sich mit den gewählten Parametern für Normalgehör Werte von D, MOD, RHS, die auf einer Geraden liegen (Fig. 2). Es wären aber auch andere Modulationsfrequenzen möglich, wenn man berücksichtigt, daß der zu untersuchende Effekt sich nicht ausschließlich bei 14 Hz, sondern auch noch bei höheren und niedrigeren Frequenzen einstellt. Daraus ergeben sich wegen der Vorverdeckung und der Nachverdeckung beim Gehör als günstige Werte solche, die zwischen 4 Hz und 100 Hz liegen.

Für den Betriebszustand Dauerrauschen D wird der 14 Hz-Generator 36 vom Modulator 35 abgetrennt, so daß unbeeinflußtes Dauerrauschen entsteht. Der maskierende Schall für D oder MOD gelangt über einen Verstärker 37, Teiler 38, Audiometer-Entzerrer 39 und den Summationspunkt 28 auf dieselbe Kopfhörermuschel 29 wie der Testschall aus dem Kanal 5. Der durch Druck auf die Taste 15 gesteuerte Teiler 38 ist wie der Teiler 26 mit einer aus der Audiometrie bekannten Anzeige versehen und erlaubt, Schalldruckpegel zwischen 0 dB und 170 dB in 10 dB-Stufen für das maskierende Rauschen einzustellen. Die Zeitfunktionen der im Kanal 6 entstehenden maskierenden Schallpegel sind für Dauerrauschen D und moduliertes Rauschen MOD rechts neben dem Kanal 6 dargestellt und mit 8 bzw. 1b bezeichnet.

Als Vertäubungsrauschen kann im mit 7 bezeichneten dritten Kanal das zur Testtonfrequenz aus dem Generator 2 und dem Kanal 5 gehörende Dauerrauschen auf dem Generator 33 benutzt werden, das am Ausgang des Filters 34 abgenommen wird. Es gelangt über einen Verstärker 40, einen Teiler 41 und einen Entzerrer 42 auf die andere Muschel 43 eines einen Kopfbügel 44 aufweisenden Kopfhörers. Über einen Druck auf die Taste 16 kann der Pegel $L_V$ des Vertäubungsrauschens in 10 dB-Stufen zwischen 20 dB und 80 dB verändert werden. Dies reicht aus, weil damit in allen Fällen der gewählten Einstellung der Parameter ausreichend vertäubt werden kann. Der jeweils erreichte Pegel $L_V$ kann ebenfalls in bei Audiometern üblicher Weise mittels Leuchtdioden angezeigt werden.

In dem Gerät werden die drei Messungen zur Erstellung von Diagrammen (Geraden) gemäß Fig. 2 wegen dem in der Reihenfolge RHS, D und MOD steigenden Schwierigkeitsgrads zweckmäßig in unten stehender Reihenfolge bei der jeweils gewählten Frequenz durchgeführt.

1. RHS Bestimmung der Ruhehörschwelle des unterbrochenen Dauertones.

2. D Bestimmung der Mithörschwelle desselben Tones, verdeckt durch Daueroktav(-tiefpaß-)rauschen mit einem Pegel, der etwa 40 dB über dem Pegel der RHS liegt. Die Erhöhung des Pegels um ca. 40 dB hat sich als günstig erwiesen. Wenn der Hörverlust größer ist als z.B. 60 dB, können auch 30 dB ausreichen.

3. MODBestimmung der Mithörschwelle desselben Tones, verdeckt durch dasselbe Rauschen, das jedoch mit 14 Hz rechteckförmig amplitudenmoduliert ist. Dieser Wert, der sich wegen der Verdeckungseigenschaften des Gehörs unter dem Gesichtspunkt einer einfachen Darstellung der Meßergebnisse als zweckmäßig erwiesen hat, liegt für Normalhörende recht genau zwischen den Werten für RHS und D.

Bei der Durchführung der Versuche wird zunächst die Testtonfrequenz $f_T$ der Betriebsart RHS und ein Pegel $L_T$ des unterbrochenen Testtones gewählt, der für die Versuchsperson (VP) hörbar ist. Die VP wird informiert, daß sie während aller Messungen nur auf diesen Testschall zu achten und durch einen aus der Audiometrie bekannten Druckknopf 45 anzuzeigen habe, daß sie den unterbrochenen Testton hört. Dieser Knopfdruck löst an der Anzeige beim Versuchsleiter ein Signal aus, indem z.B. eine Lampe 46 aufleuchtet. Mit dieser Anzeige, die durch Veränderung des Pegels L des Testtons in 5 dB- bzw. 2,5 dB-Stufen erreicht wurde, bestimmt der Versuchsleiter die Ruhehörschwelle RHS und markiert den Wert im Diagramm z.B. durch einen Kreis.

Nun wird die Mithörschwelle bei Betriebsart Dauer D auf die gleiche Weise bestimmt. Der Pegel $L_M$ des maskierenden Rauschens wird dazu etwa 40 dB über dem Ruhehörschwellenpegel gewählt. Zur Dokumentation wird dieser Wert $L_M$ ebenfalls im Diagramm an der vorgesehenen Stelle mit einem Kreuz markiert, die Mithörschwelle D, wie oben für RHS beschrieben, gemessen und an der dafür vorgesehenen Stelle des Diagramms eingetragen. Schließlich wird ohne Veränderung des Pegels $L_M$ des maskierenden Rauschens als letzte Messung die Mithörschwelle der Betriebsartmodulation MOD bestimmt. Der Wert liegt zwischen denjenigen für RHS und D, für Normalhörende fast genau in der Mitte zwischen beiden. Die beiden letztgenannten Mithörschwellen D und MOD werden ebenfalls als Kreise im Diagramm (Fig. 2) eingetragen, so daß, wie aus Fig. 2 hervorgeht, eine durch die Meßpunkte für RHS, MOD und D gelegte Gerade als Protokoll entsteht.

Als Beispiel für die Abhängigkeit der Meßergebnisse vom Pegel $L_M$ des maskierenden Rauschens sind in Fig.5 die Protokolle einer normalhörenden VP bei den drei genannten Frequenzen 500 Hz, 1500 Hz und 4000 Hz und bei Benutzung von Pegeln $L_M$ zwischen 20 und 100 dB mit jeweils 10 dB Abstand dargestellt. Sie zeigen, daß die Mithörschwellen D in gleichem Maße wachsen wie die Pegel $L_M$ des Rauschens. Dabei liegt der Pegel $L_M$ etwa 10 dB über der Mithörschwelle D.

Die Mithörschwellen MOD für moduliertes Rauschen liegen unabhängig vom Pegel $L_M$ etwa in der Mitte zwischen den Werten für D und RHS, so daß die Verbindung der als Kreise angegebenen Meßwerte ein Geradenbüschel mit verschiedenen Steigungen ergibt, das vom Punkt RHS ausgeht.

Daraus kann entnommen werden, daß die Vorgabe des Wertes des maskierenden Rauschens $L_M$ nicht kritisch ist. Es kann vielmehr so gewählt werden, daß die VP auch bei geschädigtem Gehör das maskierende Rauschen nicht zu laut empfindet und trotzdem eine Anhebung der Ruhehörschwelle zur Mithörschwelle D von Dauerrauschen von etwa 30 dB erreicht wird. Dies ergibt sich bei einem Pegel $L_M$, der etwa 40 dB über der Ruhehörschwelle RHS liegt.

Für den Normalhörenden, d.h. für eine VP mit normalem zeitlichem Auslösungsvermögen des Gehörs, sind die gemessenen Werte in der Fig. 2 in ein Diagramm mit in gleichen Abständen auf der Abszisse eingetragenen Markierungen für RHS, MOD und D und für die Größen (dB) in Ordinate. Die unter den genannten Randbedingungen gefundenen drei als Kreise eingetragenen Meßwerte für RHS, MOD und D liegen dann recht gut auf einer Geraden, wobei nur Schwankungen von ± 2,5 dB auftreten. Diese Form der Lage der Meßwerte erlaubt eine einfache Prüfung der Funktionsfähigkeit des Gehörs im Hinblick auf das Zeitauflösungsvermögen.

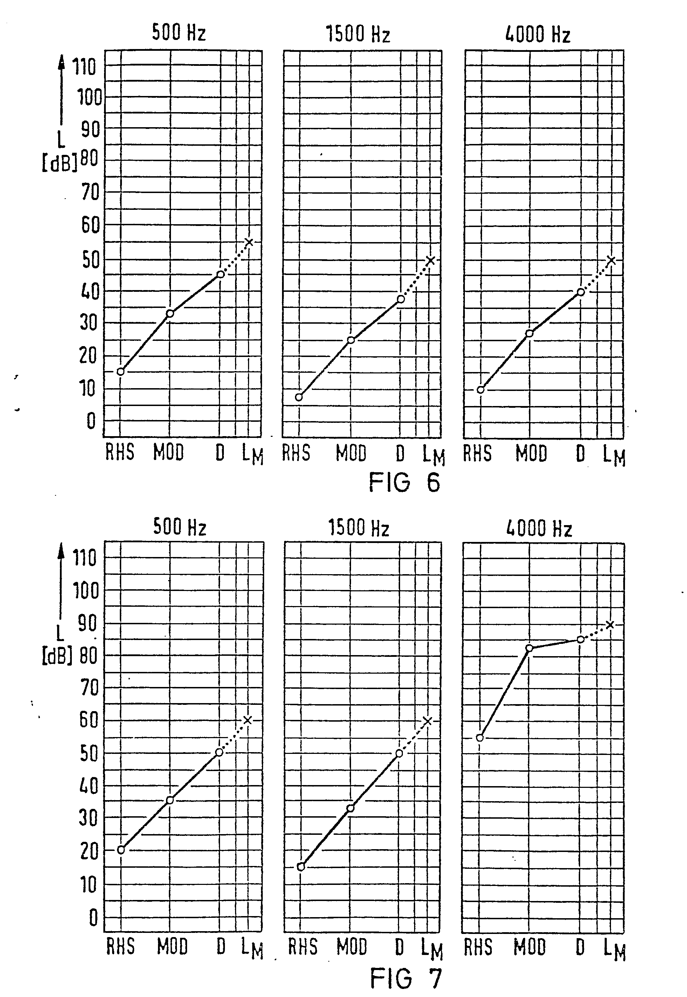

In Fig. 6 sind Zentralwerte und wahrscheinliche Schwankungen der Meßwerte von 16 normalhörenden VP aufgetragen; die dazu eingehaltenen Randbedingungen sind $L_M$ um 40 dB ± 5 dB über der RHS. Diese Daten zeigen, wie gering die Abweichungen selbst nach Mittelwertsbildungen bleiben, in die eingeht, daß zwar die RHS auf 2,5 dB genau gemessen, der Pegel $L_M$ jedoch nur auf 5 dB genau eingestellt werden kann. Die individuelle Abweichung des Wertes MOD vom exakten Mittelwert (D + RHS)/2 ergab im Mittel 2,5 dB ± 2,5 dB bei 500 Hz, 0 dB ± 1,25dB bei 1500 Hz und 2,5 dB ± 2,5 dB bei 4000 Hz. Daraus kann aber entnommen werden, daß die Beschreibungsform "wenn die im Protokoll als Kreise eingetragenen Daten auf einer Geraden liegen, entspricht dies normalem Zeitauflösungsvermögen des Gehörs" sehr gut erfüllt ist.

Als Beispiel für ein nicht normales Zeitauflösungsvermögen bei hohen Frequenzen ist in Fig. 7 das Protokoll einer VP mit einem Hörverlust von etwa 45 dB bei Frequenzen oberhalb 3 kHz wiedergegeben. Es zeigt normales Verhalten bei 500 und bei 1500 Hz, bei denen auch kein Hörverlust vorhanden ist. Bei der hohen Frequenz von 4000 Hz tritt jedoch ein abnormaler Verlauf des erhaltenen Diagrammes auf. Dort ist nicht nur die Ruhehörschwelle RHS angehoben, sondern auch die Mithörschwelle MOD ganz deutlich über die Verbindungsgerade zwischen RHS und D erhöht. Sie liegt nur wenige dB unter dem Wert für D. Dies würde im Mithörschwellen-Periodenmuster der bekannten art (Fig. 1) nur einer ganz geringen Absenkung während der Pause zwischen 1 und 1a entsprechen, in der kein Rauschen angeboten wird. Die Methode der Messung der Mithörschwellen von Testtönen, die durch Oktav-Dauerrauschen bzw. amplitudenmoduliertes Rauschen gemäß der Erfindung durchgeführt wird, ergibt dieselbe Aussage auf viel einfachere Weise.

**Patentansprüche**

1. Elektroakustisches Meßgerät mit einem Testton-Kanal (5) und einem Maskierungsschall-Kanal (6) zur Prüfung des zeitlichen Auflösungsvermögens des Gehörs mittels eines Testtones, der mittels eines Modulators (23) unterbrochen und dem modulierter Maskierungsschall über einen Schalter (12) zugefügt sein kann, wobei die Frequenzlage des Testton-Kanals und die Pegel beider Kanäle einstellbar sind und der Maskierungsschall-Kanal einen Impulsmodulator (35) enthält, dadurch gekennzeichnet, daß der dem von einem Tongenerator (22), dessen Frequenz über einen Steller wählbar ist, ausgehende Testton-Kanal (5) zuschaltbare Maskierungsschall-Kanal (6) als Generator für den Maskierungsschall einen Rauschgenerator (33) enthält mit Mitteln (34) zur Erzeugung von in ihrer Frequenz über eine Kopplung an den Steller (11) des Testton-Kanals an die Mittenfrequenz des eingestellten Testtones angleichbaren Rauschbändern und Mitteln (38) zur Anhebung des Pegels gegenüber der Ruhehörschwelle des jeweiligen Testtones und daß der Impulsmodulator (35) abschaltbar (13) ist.

2. Gerät nach Anspruch 1, gekennzeichnet durch Frequenzbandauswahlfilter als Mittel zur Angleichung der Mittenfrequenz der Rauschbänder an Änderung der Frequenz des Testtones, Mittel zur Anhebung des Pegels des Maskierungsschalls um 10 bis 100 dB gegenüber der Ruhehörschwelle des Testtones und einen abschaltbaren Impulsmodulator von 5 bis 100 Hz.

3. Gerät nach Anspruch 2, dadurch gekennzeichnet, daß die Filter Oktav-Bandpässe und ein Tiefpaß sind.

4. Gerät nach Anspruch 1, 2 und/oder 3, dadurch gekennzeichnet, daß der Rauschgenerator ein solcher für weißes Rauschen ist.

5. Gerät nach einem der vorhergehenden Ansprüche, dadurch gekennzeichnet, daß ein zusätzlicher Vertäubungsschall-Kanal vorgesehen ist, der Mittel umfaßt zum Anschluß an einen zweiten Hörer, der mit dem, der den Testschall und den Maskierungsschall überträgt, mittels eines Kopfbügels zu einem Kopfhörerpaar vereinigt ist.

6. Gerät nach einem der vorhergehenden Ansprüche, dadurch gekennzeichnet, daß die einstellbaren Frequenzen bzw. Mittenfrequenzen im Bereich von 250 bis 6000, vorzugsweise 500 bis 4000 Hz, liegen.

7. Gerät nach Anspruch 1, dadurch gekennzeichnet, daß im Testton-Kanal ein Unterbrecher vorgesehen ist, der 500 ms ein- und 500 ms ausschaltet.

8. Gerät nach Anspruch 1, dadurch gekennzeichnet, daß die Frequenzen und Mittenfrequenzen der Generatoren für den Test- und Maskierungsschall umschaltbar auf 500 Hz, 1500 Hz und 4000 Hz einstellbar sind.

9. Gerät nach anspruch 1, dadurch gekennzeichnet, daß die Schallkanäle Schaltmittel zur schrittweisen Veränderung des Schalldruckpegels enthalten.

10. Gerät nach Anspruch 9, dadurch gekennzeichnet, daß die Pegelsteller in Schritten von 2,5 bis 10 dB im Bereich von -5 bis 115 dB schaltbar sind.

11. Gerät nach Anspruch 9, dadurch gekennzeichnet, daß im Testton-Kanal Schaltmittel vorgesehen sind zur wahlweisen Absenkung und/oder Anhebung des in Schritten veränderbaren Schalldruckpegels.

12. Gerät nach den Ansprüchen 10 und 11, dadurch gekennzeichnet, daß die Schritte 5 dB und die Absenkung und/oder Anhebung 2,5 dB betragen.

13. Gerät nach Anspruch 1, dadurch gekennzeichnet, daß die Pegelsteller im Maskierungsschall- und Vertäubungsschall-Kanal in 10 dB-Schritten zwischen 0 und 110 dB schaltbar sind.

14. Gerät nach Anspruch 1, dadurch gekennzeichnet, daß im Maskierungsschall-Kanal eine Modulation mit 14 Hz erfolgt, so daß die Perioden 72 ms und Pausen von ca. 36 ms entstehen.

15. Gerät nach Anspruch 1, dadurch gekennzeichnet, daß die Modulationsfrequenz variabel ist.

**Claims**

1. Electro-acoustic measuring device comprising a test tone channel (5) and a masking sound channel (6) in order to test the time-wise acoustic resolving capacity by means of a test tone, which can be interrupted by means of a modulator (23) and added to the modulated masking sound by means of a switch ( 12), where the frequency position of the test tone channel and the levels of both channels are adjustable and the masking sound channel comprises a pulse modulator (35), characterised in that the masking sound channel (6) which can be connected to the test tone channel (5), which emanates from a tone generator (22), whose frequency is selectable by means of a regulating unit (11), comprises a noise generator ( 33) as a generator for the masking sound, which generator ( 33) comprises means (34) for producing noise bands which in their frequency can be adapted to the centre frequency of the adjusted test tone by means of a coupling to the regulating unit (11) of the test tone channel, and comprising means (38) for increasing the level relative to the aural no-sound threshold of the respective test tone, and that the pulse modulator (35) can be switched off (13).

2. A device as claimed in claim 1, <u>characterised by</u> frequency band selector filters as means for adapting the centre frequency of the noise bands to the change of the frequency of the test tone, means for increasing the level of the masking sound by 10 to 100 dB in relation to the aural no-sound threshold of the test zone and a pulse modulator of 5 to 100 Hz which can be switched off.

3. A device as claimed in claim 2, <u>characterised in</u> that the filters are octave band pass filters and a low-pass filter.

4. A device as claimed in claims 1, 2 and/or 3, <u>characterised in</u> that the noise generator is one for white background noise.

5. A device as claimed in one of the preceding claims, <u>characterised in</u> that there is arranged an additional deafening sound channel which comprises means for the connection to a second earphone which is connected to the earphone which transmits the test sound and the masking sound, to form a pair of earphones by means of a head clip.

6. A device as claimed in one of the preceding claims, <u>characterised in</u> that the adjustable frequencies or centre frequencies lie in the region of 250 to 6000, preferably 500 to 4000 Hz.

7. A device as claimed in claim 1, <u>characterised in</u> that an interrupter which switches on for 500 ms and switches off for 500 ms is arranged in the test tone channel.

8. A device as claimed in claim 1, <u>characterised in</u> that the frequencies and centre frequencies of the generators for the test and masking sound are adjustable so as to be capable of being switched over to 500 Hz, 1500 Hz and 4000 Hz.

9. A device as claimed in claim 1, <u>characterised in</u> that the sound channels comprise switching means for the gradual change of the sound intensity level.

10. A device as claimed in claim 9, <u>characterised in</u> that the level regulators can be switched in steps of 2.5 to 10 dB in the region of -5 to 115 dB.

11. A device as claimed in claim 9, <u>characterised in</u> that switching means for alternatively decreasing and/or increasing the sound intensity level, which is gradually adjustable, are arranged in the test tone channel.

12. A device as claimed in claims 10 and 11, <u>characterised in</u> that the steps amount to 5 dB and the decrease and/or increase 2.5 dB.

13. A device as claimed in claim 1, <u>characterised in</u> that the level regulators can be switched in the masking sound and deafening sound channel in 10 dB steps betweeen 0 and 110 dB.

14. A device as claimed in claim 1, <u>characterised in</u> that a modulation by means of 14 Hz is effected in the masking sound channel, so that periods of 72 ms and pauses of approximately 36 ms occur.

15. A device as claimed in claim 1, <u>characterised in</u> that the modulation frequency is variable.

**Revendications**

1. Appareil de mesure électro-acoustique comportant un canal (5) de transmission d'un son d'essai et un canal (6) de transmission d'un son de masquage pour le contrôle du pouvoir de résolution dans le temps de l'ouïe au moyen d'un son d'essai, qui peut être interrompu au moyen d'un modulateur (23) et être ajouté, par l'intermédiaire d'un commutateur (12), au son de masquage modulé, et dans lequel la position de fréquence du canal de transmission du son d'essai et le niveau des deux canaux sont réglables et le canal de transmission du son de masquage contient un modulateur d'impulsions (35), caractérisé par le fait que le canal (6) de transmission du son de masquage, qui peut être raccordé au canal (5) de transmission du son d'essai partant d'un générateur acoustique (22), dont la fréquence peut être sélectionnée au moyen d'un régulateur (11), contient, en tant que générateur pour le son de masquage, un générateur de bruit (33), avec des moyens (34) pour produire des bandes de bruit, dont la fréquence peut être réglée par l'intermédiaire d'un couplage avec le régulateur (11) du canal de transmission du son d'essai, sur la fréquence centrale du son d'essai réglé, et des moyens (38) pour relever le niveau par rapport au seuil d'écoute au repos du son d'essai respectif et que le modulateur d'impulsions (35) est débranchable.

2. Appareil suivant la revendication 1, caractérisé par un filtre de sélection de bandes de fréquences en tant que moyen pour adapter la fréquence centrale des bandes de bruit sur la base d'une variation de la fréquence du son d'essai, des moyens pour accroître le niveau du son de masquage de 10 à 100 dB par rapport au seuil d'écoute au repos du son d'essai, et un modulateur d'impulsions débranchable réalisant une modulation entre 5 et 100 Hz.

3. Appareil suivant la revendication 2, caractérisé par le fait que les filtres sont des filtres passe-bande d'octaves et un filtre passe-bas.

4. Appareil suivant la revendication 1, 2 et/ou 3, caractérisé par le fait que le générateur de bruit est un générateur produisant un bruit blanc.

5. Appareil suivant l'une des revendications précédentes, caractérisé par le fait qu'il est prévu un canal supplémentaire de transmission d'un son d'assourdissement, qui comporte des moyens permettant le raccordement à un second écouteur qui est réuni à l'écouteur, qui transmet le son d'essai et le son de masquage, au moyen d'un serre-tête pour former un couple d'écouteurs.

6. Appareil suivant l'une des revendications précédentes, caractérisé par le fait que les fréquences ou les fréquences centrales réglables sont situées dans la plage comprise entre 250 et 6000 et de préférence entre 500 et 4000 Hz.

7. Appareil suivant la revendication 1, caractérisé par le fait que dans le canal de transmission du son d'essai, il est prévu un interrupteur qui est branché pendant 500 ms et est débranché pendant 500 ms.

8. Appareil suivant la revendication 1, caractérisé par le fait que les fréquences et les fréquences centrales des générateurs pour le son d'essai et le son de masquage sont réglables de manière à pouvoir être commutées sur 500 Hz, 1500 Hz et 4000 Hz.

9. Appareil suivant la revendication 1, caractérisé par le fait que les canaux de transmission du son contiennent des moyens de commutation permettant de modifier par échelons le niveau de pression acoustique.

10. Appareil suivant la revendication 9, caractérisé par le fait que les régulateurs de niveau peuvent être commutés selon des échelons de 2,5 à 10 dB dans la plage de - 5 à 115 dB.

11. Appareil suivant la revendication 9, caractérisé par le fait qu'il est prévu, dans le canal de transmission du son d'essai, des moyens permettant de réduire et/ou d'accroître le niveau de pression acoustique modifiable par échelons.

12. Appareil suivant les revendications 10 et 11, caractérisé par le fait que les échelons ont une valeur de 5 dB, et/ou l'accroissement est égal à 2,5 dB.

13. Appareil suivant la revendication 1, caractérisé par le fait que les régulateurs de niveau situés dans le canal de transmission du son de masquage et dans le canal de transmission du son d'assourdissement sont commutables par échelons de 10 dB entre 0 et 110 dB.

14. Appareil suivant la revendication 1, caractérisé par le fait qu'une modulation à 14 Hz est réalisée dans le canal de transmission du son de masquage de sorte qu'il apparaît des périodes de 72 ms et des pauses d'environ 36 ms.

15. Appareil suivant la revendication 1, caractérisé par le fait que la fréquence de modulation est variable.

FIG 1

FIG 2

FIG 3

FIG 4

FIG 5

FIG 6

FIG 7